# EUROPÄISCHE PATENTSCHRIFT

(11) **EP 0 401 568 B1**
(45) Veröffentlichungstag und Bekanntmachung des Hinweises auf die Patenterteilung: **16.03.1994**
(21) Anmeldenummer: 90109307.0
(22) Anmeldetag: 17.05.1990
(51) Int. Cl.: A61L 2/26, A61B 1/12

(54) **Sterilisationsbehälter für Instrumente**
Container for sterilising instruments
Récipient pour la stérilisation d'instruments

(30) Priorität: 03.06.1989 DE 3918147
(43) Veröffentlichungstag der Anmeldung: 12.12.1990
(73) Patentinhaber: RIWOPLAN MEDIZIN-TECHNISCHE EINRICHTUNSGESELLSCHAFT MBH, D-75438 Knittlingen (DE)
(72) Erfinder: Mönch, Harry, D-7134 Knittlingen (DE)
(74) Vertreter: Wilcken, Thomas, Dipl.-Ing.

(56) Entgegenhaltungen:
- DE-B- 2 929 227
- DE-C- 3 833 998

## Beschreibung

Die Erfindung geht aus von einem aus einer Drahtkonstruktion bestehenden Behälter zur Aufnahme von Instrumenten, insbesondere von Endoskopen bzw. Teilen derselben, zum Zwecke der Sterilisation oder Desinfektion, umfassend ein Unterteil und ein lösbar auf diesem festlegbares Oberteil.

Ein solcher Behälter ist in der DE-B-29 29 227 beschrieben. Das Unterteil des Behälters besteht aus einem Drahtboden, der von vier netzartig ausgebildeten Seitenwänden umgeben ist, die am Außenrahmen des Drahtbodens befestigt sind. Der flache Deckel des Behälters besteht aus einem umlaufenden Rahmen, an dem ein flaches Drahtnetz als Wandteil befestigt ist. Während die Übergänge zwischen den Seitenwänden von Unterteil und Deckel jeweils gerundet sind, sind die Übergänge zwischen dem Drahtboden und den zugehörigen Seitenwänden des Unterteils scharfkantig. Bei diesem Behälter ist es nachteilig, daß einerseits das Einbringen dieser Behälter in eine aus einer Folie gefertigte, schlauchförmig ausgebildete Verpackung nur sehr umständlich erfolgen kann und andererseits die bei diesen Behältern vorhandenen scharfen Kanten eine Beschädigung der Folie bewirken, wodurch die Sterilität des verpackten Instrumentes nicht mehr gewährleistet ist.

Ein weiterer Behälter ist in der DE-A-34 36 489 offenbart. Dieser Behälter weist an seiner Oberseite und an seiner Unterseite eine Vielzahl von Durchbrüchen auf, um einerseits dem zur Sterilisation verwendeten Gas freien Zutritt in den sonst geschlossenen Behälterinnenraum zu ermöglichen und andererseits die nach der Sterilisation erforderliche Auslüftung zu gewährleisten, ohne daß dazu der Behälter geöffnet werden muß. Des weiteren sind Metallbehälter in Gebrauch, die im Bereich der Gas-Eintrittsöffnung und Gas-Austrittsöffnung ein Filtertuch ausweisen. Die Instrumente selbst werden zur Vermeidung einer unbeabsichtigten Kontamination und von Beschädigungen durch sogenannte Falttucheinsätze geschützt. Derartige Sterilisationgsbehälter sind aus dem Katalogblatt AR 40/II.80 der Patentinhaberin zu entnehmen.

Der diesen Behältern anhaftende Nachteil besteht darin, daß, bedingt durch den relativ geringen freien Gasdurchtritt einerseits und durch die verwendeten Falttucheinsätze andererseits, die erforderliche Auslüftung unter Umständen erhebliche Zeit in Anspruch nimmt und daß auch die Bildung von Kondenswasser nicht ausgeschlossen werden kann, was auf den großflächigen Metallanteil zurückzuführen ist.

Die der Erfindung zugrundeliegende Aufgabe besteht darin, einen Behälter der einleitend angeführten Art so zu verbessern, daß die darin befindlichen Instrumente innerhalb kürzester Zeit vollständig auslüften können, die Bildung von Kondenswasser innerhalb des Behälters vermieden wird sowie die Voraussetzungen für eine einfachere Verpackung verbessert sind und Beschädigungen derselben vermieden werden.

Die Lösung dieser Aufgabe ist in dem Patentanspruch 1 angeführt.

Durch die erfindungsgemäße Ausbildung des Behälters ist gewährleistet, daß das zu sterilisierende oder zu desinfizierende Instrument im erforderlichen Umfang von dem bei der Gassterilisation verwendeten Gas bzw. bei der Desinfektion von dem verwendeten flüssigen Desinfektionsmittel umspült wird und im Anschluß daran eine optimale Entlüftung erfolgen kann. Durch die linienförmige Anlage der Instrumententeile und Aufnahmeelemente aneinander und durch die Ausbildung des Behälters in Form eines Käfigs wird bei der Durchführung einer Desinfektion in einem flüssigen Desinfektionsmittel sowohl ein unbeabsichtigtes Zurückbleiben eines Teiles des flüssigen Desinfektionsmittels als auch die Ansammlung von Kondenswasser innerhalb des Behälters wirksam vermieden. Die jeweils zum endseitigen Behälterende hin abfallenden Abdeckungen ermöglichen ein einfaches Einführen des Behälters in eine schlauchförmige Folienverpackung zur sterilen Aufbewahrung des mit den Instrumenten versehenen Behälters. Auch weist der Draht-Behälter keinerlei scharfe Kanten mehr auf, so daß Beschädigungen der Verpackungsfolie vermieden werden.

In weiterer Ausbildung des erfindungsgemäßen Behälters kann das Unterteil mit Stützfüßen und die Abdeckkappen mit Stütztaschen einer der der Stützfüße entsprechenden Anordnung versehen sein. Dabei können die Stütztaschen jeweils in der geneigten Fläche der Abdeckkappen angeordnet sein und jeweils eine Stützfläche aufweisen, wobei alle Stützflächen in einer gemeinsamen Ebene ausgerichtet sind, so daß mehrere Behälter bei sicherem Stand aufeinander stapelbar sind.

In besonders zweckmäßiger Ausführung kann vorgesehen sein, daß das Unterteil wie auch das Oberteil aus einer Vielzahl von solchen Drahtformteilen aufgebaut ist, deren jedes aus einem Draht bestimmter Länge besteht, dessen beide Endbereiche unter Bildung eines Halbkreises in gemeinsamer Ebene umgebogen sind, so daß deren Enden bis zur gegenseitigen Berührung aufeinander zuweisen, in welcher Lage die Enden durch Löten oder Schweißen miteinander verbunden sind, um so ein Endlos-Drahtelement zu bilden.

Zur Formgebung von Unterteil und Oberteil des Behälters können diese Drahtformteile durch rechtwinkeliges Abbiegen der jeweils den halbkreisförmigen Teil umfassenden Endbereiche U-förmig gestaltet sein, so daß das Unterteil und das Oberteil durch in Behälterlängsrichtung sowie durch spantenartig quer dazu angeordnete Drahtformteile gebildet werden, wobei die Kreuzungspunkte durch Löten oder Schweißen unlösbar verbunden sind.

Die dabei aufragenden halbkreisförmigen Endbereiche können jeweils mittels eines Drahtreifes durch Löten oder Schweißen verbunden sein, wobei jeder derselben entsprechend der Form der Behälteröffnung als rechteckförmiges Endlos-Drahtelement ausgebildet sein kann.

Das Oberteil kann auf dem Unterteil dadurch deckungsgleich geführt werden, daß die Abdeckkappen fest mit dem Oberteil verbunden sind und den Drahtreif des Unterteiles überfassende Begrenzungsflächen aufweisen, wobei die lösbare Verbindung von Unterteil und Oberteil durch Federelemente erfolgen kann.

Zwecks unverrückbarer Fixierung der zu behandelnden Instrumente können an deren Bauform angepaßte Aufnahmeelemente vorgesehen sein, die sich in Schließstellung des Behälters einander teilweise übergreifend an dem Unterteil bzw. dem Oberteil durch Befestigungselemente lösbar befestigt sind. Dabei kann ein sicherer Halt der Befestigungselemente an den Drahtformteilen durch eine Klemm- und formschlüssige Verbindung erfolgen.

Der erfindungsgemäße Desinfektionsbehälter wird nachfolgend anhand eines in der Zeichnung dargestellten Ausführungsbeispieles näher erläutert. Es zeigen:
- Figur 1: den erfindungsgemäß ausgebildeten Desinfektionsbehälter in Draufsicht in einer ersten Ausführungsform,
- Figur 2: eine Seitenansicht des Desinfektionsbehälters nach Figur 1 mit eingelagerter Schnittdarstellung entlang der Linie II-II nach Figur 1,
- Figur 3: eine Schnittdarstellung entlang der Linie III-III nach Figur 2,
- Figur 4: eine Schnittdarstellung entlang der Linie IV-IV nach Figur 2,
- Figur 5: eine Seitenansicht des erfindungsgemäßen Desinfektionsbehälters in einer zweiten Ausführungsform mit teilweise geschnittener Abdeckkappe,
- Figur 6: eine teilweise dargestellte Seitenansicht nach Figur 1 mit Schnittdarstellungen entlang der Linie II-II einer zweiten Ausführungsform,
- Figuren 7,8: Schnittdarstellungen durch die Befestigungselemente zum Festlegen der Aufnahmeelemente für die Instrumententeile,
- Figur 9: eine teilweise dargestellte Ansicht von unten auf den erfindungsgemäßen Desinfektionsbehälter.

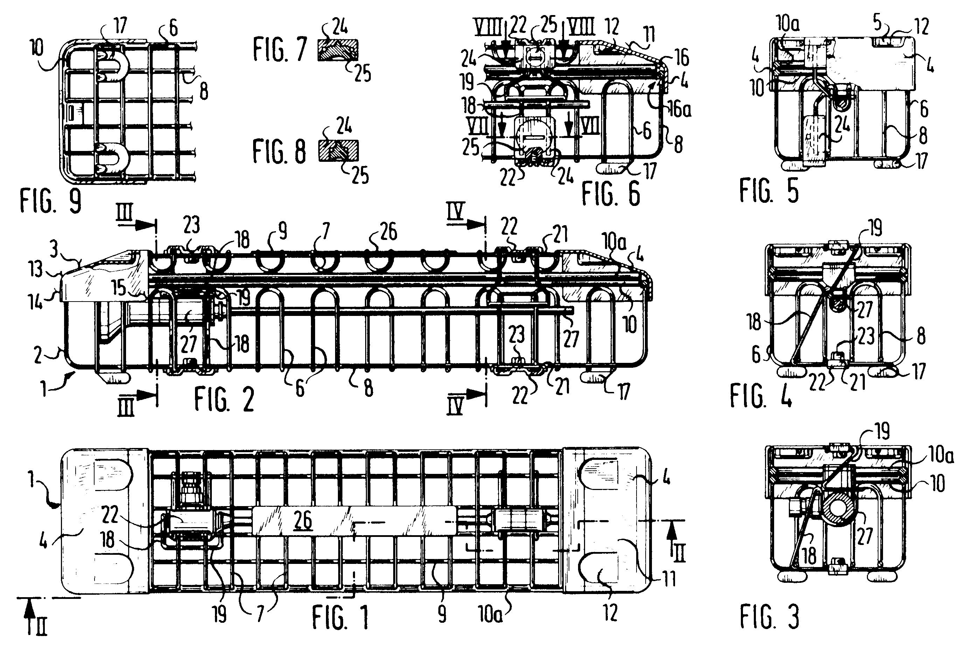

Der erfindungsgemäß ausgebildete Desinfektionsbehälter 1 ist in den Figuren 1 und 2 mit einer darin zur Durchführung der Desinfektion lösbar festgelegten Endoskopoptik dargestellt und besteht im wesentlichen aus einem die Optik bzw. das zu desinfizierende Instrument aufnehmenden Unterteil 2 und einem auf diesem lösbar festlegbaren, das Unterteil 2 abdeckenden Oberteil 3, das an seinen beiden Enden jeweils eine vollflächig ausgebildete Abdeckkappe 4 mit jeweils zwei eingeprägten Stützflächen 5 aufweist. Das Unterteil 2 wie auch das Oberteil 3 bestehen aus einer Vielzahl von im Abstand zueinander angeordneten und unlösbar auf mehreren in Längsrichtung des Behälters 1 ausgerichteten Drahtformteilen 8 bzw. 9 angebrachten Drahtformteilen 6 bzw. 7. Diese bestehen jeweils aus einem Draht bestimmter Länge, dessen beide Endbereiche unter Bildung eines Halbkreises in gemeinsamer Ebene umgebogen sind, so daß deren Enden bis zur gegenseitigen Berührung aufeinander zuweisen, in welcher Lage ihre Endflächen durch Löten oder Schweißen miteinander verbunden sind, um so ein Endlos-Drahtelement zu bilden. Zur Nachbildung der Seitenteile von Unterteil 2 und Oberteil 3 sind beide Enden der länglich ovalen Drahtelemente im rechten Winkel abgebogen, so daß jeweils ein U-förmiges Endlos-Drahtelement entsteht. Die aufragenden halbkreisförmigen Endbereiche der Endlos-Drahtelemente 6 und 8 bzw. 7 und 9 sind jeweils mit einem Drahtreif 10 bzw. 10a unlösbar verbunden, welche jeweils die Umrahmung der Behälteröffnung von Unterteil 2 und Oberteil 3 bilden und entsprechend der Behälterform als rechteckförmige Endlos-Drahtelemente deckungsgleicher Formgebung ausgebildet sind.

Die Abdeckkappen 4 bilden jeweils den endseitigen Abschluß des Oberteiles 3 und sind jeweils mit dem Drahtformteil 9 und dem Drahtreif 10a unlösbar verbunden. Die Stütztaschen 5 sind in zu den Endbereichen des Oberteiles 3 geneigt verlaufenden Flächen 11 der Abdeckkappen 4 eingeprägt und bilden in einer gemeinsamen Ebene angeordnete Stützflächen 12 aus. Die Abdeckkappen 4 sind mit Begrenzungsflächen 14 versehen, die den Drahtreif 10a seitlich und endseitig umfassen und die entsprechenden Teile des Drahtreifs 10 des Oberteiles 3 gegenüber dem Unterteil 2 deckungsgleich zentrierend überfassen. Die Fläche 11 jeder Abdeckkappe 4 geht über einen Radius 13 in die endseitige Begrenzungsfläche 14 über. Ebenso schließen die seitlichen Teile derselben mit einem Radius 15 ab, um Verletzungen, Beschädigungen oder dergleichen hervorrufende Ecken zu vermeiden.

Die lösbare Verbindung des Oberteiles 3 mit dem Unterteil 2 erfolgt mittels an den Abdeckkappen 4 angebrachten Federelementen 16, die den Drahtreif 10 im endseitigen Bereich mit einer Nase 16a übergreifen.

Das Unterteil 2 ist an seinem die Bodenfläche bildenden Seitenteil mit vier Stützfüßen 17 bestückt, deren Anordnung mit der der Stütztaschen 5 abgestimmt ist, so daß die Stützfüße 17 auf den Stützflächen 12 der Stütztaschen 5 zu stehen kommen, wenn zwei Behälter 1 aufeinander gestapelt werden.

Die Aufnahme und lösbare Festlegung zum Beispiel einer Endoskopoptik 27 bzw. von Instrumenten allgemein erfolgt durch mehrere an dem Unterteil 2 und dem Oberteil 3 befestigbaren Aufnahmeelementen 18 bzw. 19, die sich teilweise übergreifend die Optik 27 zwischen sich einschließen und spielfrei in der jeweiligen Lage fixieren. Die Aufnahmeelemente 18 und 19 sind mittels Befestigungselementen aus jeweils zwei durch eine Schraubverbindung 23 gegeneinander verspannbaren Klemmteilen 21 und 22 festlegbar. Zu diesem Zweck werden jeweils die beiden parallel zueinander verlaufenden Teile der Drahtformteile 6 bzw. 7 und die freien Endteile der Aufnahmeelemente 18 bzw. 19 zwischen den beiden Klemmteilen 21 und 22 eingeklemmt. Alternativ können die Elemente 18 und 19 auch mit den Teilen 2 und 3 punktverschweißt sein.

Hinweise auf das Datum der Sterilisation und dergleichen können auf dem Kennzeichnungsfeld 26 an der Oberseite des Oberteiles 3 des Sterilisationsbehälters 1 handschriftlich vermerkt werden.

In weiterer Ausbildung des erfindungsgemäßen Sterilisationsbehälters 1 ist es denkbar, anstelle von U-förmig ausgebildeten Drahtformteilen Stanzteile mit gleicher Raumform zu verwenden oder aber den Behälter aus einem Metallgitter zu fertigen, wobei lediglich bestimmte Flächenbereiche zur Bildung der beiden senkrecht zur Bodenfläche angeordneten Stirn- und Seitenteilen umgebogen werden müssen. Weiterhin ist es denkbar, die beiden mittels einer Schraubverbindung miteinander verspannten und die jeweiligen Schenkel der Drahtformteile und der Aufnahmeelemente einschließenden Klemmteile 21, 22 durch Befestigungsmittel entsprechend den Figuren 7 und 8 zu ersetzen, wobei diese aus einer die genannten Teile aufnehmenden, ein Gewinde aufweisenden Aufnahme 24 und einer als Spannelement ausgebildeten Schraube 25 bestehen können. Zur Festlegung der Aufnahme 24 auf dem entsprechenden Drahtformteil einerseits und zur Fixierung des Aufnahmeelementes 18 bzw. 19 andererseits kann die Aufnahme 24 vorteilhafterweise einstückig als Montagewinkel ausgebildet sein.

## Patentansprüche

1. Aus einer Drahtkonstruktion bestehender Behälter zur Aufnahme von Instrumenten, insbesondere Endoskopen bzw. Teilen derselben, zum Zwecke der Sterilisation oder Desinfektion, umfassend ein Unterteil und ein lösbar auf diesem festlegbares Oberteil, dadurch gekennzeichnet, daß die Übergänge von sämtlichen, den Behälterraum umschließenden Begrenzungsflächen des Unterteils (2) und des Oberteils (3) gerundet ausgebildet sind und daß die beiden endseitigen Abschnitte der oberen Begrenzungsfläche des Oberteiles (3) in Richtung zum jeweiligen Behälterende hin und zu dem Unterteil (2) geneigt (11) verlaufen.

2. Behälter nach Anspruch 1, dadurch gekennzeichnet, daß das Oberteil (3) in seinen Endbereichen mit Abdeckkappen (4) versehen ist, die jeweils eine zu den Endbereichen hin geneigte Fläche (11) aufweisen.

3. Behälter nach Anspruch 1 oder 2, dadurch gekennzeichnet, daß das Unterteil (2) mit Stützfüßen (17) und die Abdeckkappen (4) mit Stütztaschen (5) einer der der Stützfüße (17) entsprechenden Anordnung versehen sind.

4. Behälter nach Anspruch 3, dadurch gekennzeichnet, daß die Stütztaschen (5) jeweils in der geneigten Fläche (11) der Abdeckkappen (4) angeordnet sind und jeweils eine Stützfläche (12) aufweisen, wobei alle Stützflächen (12) in einer gemeinsamen Ebene ausgerichtet sind.

5. Behälter nach einem der Ansprüche 1 bis 4, dadurch gekennzeichnet, daß das Unterteil (2) wie auch das Oberteil (3) aus einer Vielzahl von Drahtformteilen (6 und 8 bzw. 7 und 9) aufgebaut ist, deren jedes aus einem Draht bestimmter Länge besteht, dessen beide Endbereiche unter Bildung eines Halbkreises in gemeinsamer Ebene umgebogen sind, so daß deren Enden bis zur gegenseitigen Berührung aufeinander zuweisen, in welcher Lage ihre Endflächen durch Löten oder Schweißen miteinander verbunden sind, um so ein Endlos-Drahtelement zu bilden.

6. Behälter nach Anspruch 5, dadurch gekennzeichnet, daß die Drahtformteile (6 bis 9) durch rechtwinkeliges Abbiegen der jeweils den halbkreisförmigen Teil umfassenden Endbereiche U-förmig gestaltet sind.

7. Behälter nach einem der Ansprüche 1 bis 6, dadurch gekennzeichnet, daß dessen Unterteil (2) und Oberteil (3) durch in Behälterlängsrichtung sowie durch spantenartig quer dazu angeordnete Drahtformteile (8 bzw. 6 und 9 bzw. 7) gebildet werden, wobei die Kreuzungspunkte durch Löten oder Schweißen unlösbar verbunden sind.

8. Behälter nach Anspruch 7, dadurch gekennzeichnet, daß die aufragenden halbkreisförmigen Endbereiche jeweils mittels eines Drahtreifes (10 bzw. 10a) durch Löten oder Schweißen verbunden sind, wobei jeder derselben entsprechend der Form der Behälteröffnung als rechteckförmiges Endlos-Drahtelement ausgebildet ist.

9. Behälter nach einem der Ansprüche 1 bis 8, dadurch gekennzeichnet, daß die Abdeckkappen (4) fest mit dem Oberteil (3) verbunden sind und den Drahtreif (10a) überfassende Begrenzungsflächen (14) aufweisen.

10. Behälter nach einem der Ansprüche 1 bis 9, dadurch gekennzeichnet, daß die lösbare Verbindung von Unterteil (2) und Oberteil (3) durch Federelemente (16) erfolgt.

11. Behälter nach einem der Ansprüche 1 bis 10, dadurch gekennzeichnet, daß an dem Unterteil (2) und dem Oberteil (3) für die Halterung der zu behandelnden Instrumente an deren Bauform angepaßte Aufnahmeelemente (18 bzw. 19) vorgesehen sind, die sich in Schließstellung des Behälters einander teilweise übergreifend an dem Unterteil (2) bzw. dem Oberteil (3) durch Befestigungselemente (21,22) lösbar befestigt sind.

12. Behälter nach Anspruch 11, dadurch gekennzeichnet, daß die Befestigungselemente (21,22) mit den Drahtformteilen (6,7bzw.8,9) eine Klemm- und formschlüssige Verbindung eingehen.

## Claims

1. A container, consisting of a wire structure, to hold instruments, in particular endoscopes or parts thereof, for the purpose of sterilising or disinfecting, comprising a lower part and an upper part, able to be secured thereon in a detachable manner, characterised in that the transitions of all the faces of the lower part (2) and of the upper part (3) surrounding the container space are constructed so as to be rounded and that the two sections of the upper face of the upper part (3) on the end side extend in the direction towards the respective container end and inclined (11) towards the lower part (2).

2. A container according to Claim 1, characterised in that the upper part (3) is provided in its end regions with cover caps (4), which in each case have a surface (11) inclined towards the end regions.

3. A container according to Claim 1 or 2, characterised in that the lower part (2) is provided with support feet (17) and the cover caps (4) are provided with support pockets (5) of an arrangement corresponding to that of the support feet (17).

4. A container according to Claim 3, characterised in that the support pockets (5) are arranged in each case in the inclined surface (11) of the cover caps (4) and in each case have a support surface (12), in which all the support surfaces (12) are aligned in a common plane.

5. A container according to one of Claims 1 to 4, characterised in that the lower part (2) and also the upper part (3) is constructed from a plurality of shaped parts of wire (6 and 8 or 7 and 9), each of which consists of a wire of particular length, the two end regions of which are bent in a common plane, forming a semi-circle, so that the ends thereof point to each other as far as mutual contact, in which position their end faces are connected with each other by soldering or welding, so as to form a continuous wire element in this way.

6. A container according to Claim 5, characterised in that the shaped parts of wire (6 to 9) are formed in a U-shape by bending at right-angles the end regions, each comprising the semicircular part.

7. A container according to one of Claims 1 to 6, characterised in that its lower part (2) and upper part (3) are formed by shaped parts of wire (8 or 6 and 9 or 7) which are arranged in longitudinal direction of the container and also in the manner of frames transversely thereto, in which the points of intersection are connected so as to be undetachable by soldering or welding.

8. A container according to Claim 7, characterised in that the rising semiciruclar end regions are connected in each case by means of a wire ring (10 or 10a) by soldering or welding, in which each of these is constructed as a rectangular endless wire element corresponding to the shape of the container opening.

9. A container according to one of Claims 1 to 8, characterised in that the cover caps (4) are firmly connected with the upper part (3) and have faces (14) engaging over the wire ring (10a).

10. A container according to one of Claims 1 to 9, characterised in that the detachable connection of lower part (2) and upper part (3) takes place by spring elements (16).

11. A container according to one of Claims 1 to 10, characterised in that on the lower part (2) and on the upper part (3), holding elements (18 or 19) are provided which, for the mounting of the instruments which are to be treated, are adapted to the structural form of the latter, and which are detachably secured, partially overlapping each other in the closed position of the container, on the lower part (2) or on the upper part (3) by fastening elements (21,22).

12. A container according to Claim 11, characterised in that the fastening elements (21,22) enter into a clamping and form locking connection with the shaped parts of wire (6,7 or 8,9).

## Revendications

1. Récipient formé par une structure en fil et servant à loger des instruments, notamment des endoscopes ou des parties d'endoscopes, pour la stérilisation ou la désinfection, comprenant une partie inférieure et une partie supérieure fixée de façon amovible sur la partie inférieure, caractérisé en ce que les jonctions entre toutes les surfaces limites, qui enserrent le compartiment du récipient, de la partie inférieure (2) et de la partie supérieure (3) sont arrondies et en ce que les deux sections d'extrémité de la surface limite supérieure de la partie supérieure (3) s'étendent en direction de l'extrémité respective du récipient (11) et sont inclinées par rapport à la partie inférieure (2).

2. Récipient selon la revendication 1, caractérisé en ce que la partie supérieure (3) comporte, dans ses zones d'extrémité, des capots de fermeture (4) qui possèdent chacun une surface inclinée (11) vers les zones d'extrémité.

3. Récipient selon la revendication 1 ou 2, caractérisé en ce que la partie inférieure (2) comporte des pieds de support (17) et les capots de fermeture (4) comportent des logements de support (5).

4. Récipient selon la revendication 3, caractérisé en ce que les logements de support (5) sont disposés respectivement dans la surface inclinée (11) du capot de fermeture (4) et possèdent respectivement une surface d'appui (12), toutes ces surfaces d'appui (12) étant alignées dans un plan commun.

5. Récipient selon l'une des revendications 1 à 4, caractérisé en ce que la partie inférieure (2) ainsi que la partie supérieure (3) sont constituées par une multiplicité de pièces de forme (6 et 8 ou 7 et 9) formées d'un fil, dont chacune est constituée par un fil de longueur déterminée, dont les deux zones d'extrémités sont repliées en formant un demi-cercle dans un plan commun de sorte que leurs extrémités sont tournées l'une vers l'autre jusqu'à se toucher mutuellement, position dans laquelle leurs surfaces d'extrémité sont reliées entre elles par brasage ou soudage de manière à former un élément de fil sans fin.

6. Récipient selon la revendication 5, caractérisé en ce que les pièces de forme (6 à 9) formées d'un fil sont conformées en forme de U par pliage à angle droit des zones d'extrémité qui enserrent respectivement la partie de forme semi-circulaire.

7. Récipient selon l'une des revendications 1 à 6, caractérisé en ce que sa partie inférieure (2) et sa partie supérieure (3) sont formées par des pièces de forme (8 ou 6) formées d'un fil, qui sont disposées dans la direction longitudinale du récipient, ainsi que par des pièces de forme (9 ou 7) formées d'un fil, qui sont disposées transversalement à la manière de membrures, les points d'intersection étant fixés de manière inamovible par brasage ou soudage.

8. Récipient selon la revendication 7, caractérisé en ce que les zones d'extrémité de forme semi-circulaire se dressant sont raccordées respectivement au moyen d'un élément de fil (10 ou 10a) par brasage ou soudage, chacune de ces zones d'extrémité étant agencée, conformément à la forme de l'ouverture du récipient, a la manière d'un élément rectangulaire formé d'un fil sans fin.

9. Récipient selon l'une des revendications 1 à 8, caractérisé en ce que les capots de fermeture (4) sont reliés de façon fixe à la partie supérieure (3) et comportent des surfaces limites (14) qui enserrent l'élément de fil (10a).

10. Récipient selon l'une des revendications 1 à 9, caractérisé en ce que la liaison amovible entre la partie inférieure (2) et la partie supérieure (3) est réalisée au moyen d'éléments de ressort (16).

11. Récipient selon l'une des revendications 1 à 10, caractérisé en ce que sur la partie inférieure (2) et sur la partie supérieure (3) sont prévus, pour la fixation des instruments à traiter, des éléments de réception (18 ou 19) adaptés à la forme de réalisation de ces instruments et qui sont fixés de façon amovible, par des éléments de fixation (21,22), à la partie inférieure (2) ou à la partie supérieure (3), en s'engageant en partie les uns au-dessus des autres, lorsque le récipient est ferme.

12. Récipient selon la revendication 11, caractérisé en ce que les éléments de fixation (21,22) établissent une liaison par serrage et par formes complémentaires avec les pièces de forme (6,7 ou 8,9) formées d'un fil.
